(19) Europäisches Patentamt
European Patent Office
Office européen des brevets

(11) Veröffentlichungsnummer : **0 335 296 B1**

# (12) EUROPÄISCHE PATENTSCHRIFT

(45) Veröffentlichungstag der Patentschrift : **15.04.92 Patentblatt 92/16**

(51) Int. Cl.$^5$ : **A61K 49/02**

(21) Anmeldenummer : **89105358.9**

(22) Anmeldetag : **25.03.89**

## (54) Tc-99m-w-Alkylphosphinico-1-hydroxyalkan-1,1-diphosphonate enthaltende Präparate zur Knochenszintigraphie sowie Verfahren zur Herstellung dieser Präparate.

(30) Priorität : **30.03.88 DE 3810819**

(43) Veröffentlichungstag der Anmeldung : **04.10.89 Patentblatt 89/40**

(45) Bekanntmachung des Hinweises auf die Patenterteilung : **15.04.92 Patentblatt 92/16**

(84) Benannte Vertragsstaaten : **AT BE CH DE ES FR GB GR IT LI LU NL SE**

(56) Entgegenhaltungen :
**EP-A- 0 096 930**
**US-A- 3 735 001**
**US-A- 4 515 766**

(56) Entgegenhaltungen :
**INTERNATIONAL JOURNAL OF APPLIED RADIATION AND ISOTOPES. vol. 33, no. 10, Oktober 1982, OXFORD GB Seiten 945 - 951; ECKELMAN W.: "In vivo chemistry of Tc99m-chelates"**

(73) Patentinhaber : **HOECHST AKTIENGESELLSCHAFT**
**Postfach 80 03 20**
**W-6230 Frankfurt am Main 80 (DE)**

(72) Erfinder : **Schwarz, Alexander, Dr.**
**Wiesenring 61**
**W-6093 Flörsheim am Main (DE)**
Erfinder : **Steinsträsser, Axel, Dr.**
**Zum Morgengraben 26**
**W-6237 Liederbach (DE)**

EP 0 335 296 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Tc-99m-ω-Alkylphosphinico-1-hydroxyalkan-1,1-diphosphonate enthaltende Präparate zur Knochenszintigraphie und Verfahren zur Herstellung dieser Präparate.

Unter den nuklearmedizinischen Verfahren hat die Skelettszintigraphie eine besondere Bedeutung erlangt, da mit ihrer Hilfe Knochenerkrankungen heute frühzeitig diagnostiziert werden können, häufig bevor sie röntgenologisch erkennbar sind. Die ersten Tc-99m-markierten osteotropen Verbindungen waren anorganische Polyphosphate, die jedoch eine relativ langsame Blutclearance aufweisen infolge ihrer Tendenz, in wäßriger Lösung zu Monophosphat zu hydrolysieren.

Die Einführung der ersten organischen Diphosphonsäure durch Yano et al. ("Tc-99m-labelled stannous ethane-1-hydroxy-1,1-diphosphonate --Tc-99m-HEDP-- : a new bone scanning agent", J. Nucl. Med. 14, 73, 1973 und US Patent 3 735 001) war ein erheblicher Fortschritt, da durch die signifikant schnellere Plasmaclearance des Tc-99m-HEDP die Zeitspanne zwischen Applikation und Beginn der Szintigraphie erheblich verkürzt werden konnte. In dieser Substanz sind beide Phosphorsäuregruppen über ein Kohlenstoffatom miteinander verknüpft, während

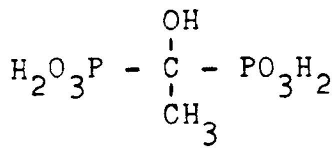

sich in den Polyphosphaten an dessen Stelle ein Sauerstoffatom befindet. In den folgenden Jahren wurde eine große Anzahl von geminalen Diphosphonsäuren beschrieben, die sich, markiert mit Technetium-99m, zur Skelettszintigraphie eignen.

Bisher haben nur drei Substanzen, Methandiphosphonsäure (MDP), 3,3-Diphosphono-1,2-propandicarbonsäure (DPD) und Hydroxymethandiphosphonsäure (HMP) in der nuklearmedizinischen Routinediagnostik eine weitverbreitete Anwendung gefunden.

Zur besseren Erkennung von Skelettläsionen, vor allem von Metastasen im Frühstadium, muß jedoch eine möglichst hohe Anreicherung des Tc-99m-Diphosphonats im gesunden Knochen nicht unbedingt von Vorteil sein. Substanzen, die im normalen Knochen nur mäßig, in der Läsion aber ausreichend gut gespeichert und außerdem rasch aus dem Blut eliminiert werden, können bei der Detailerkennbarkeit von Läsionen von Vorteil sein.

Viele bisher durchgeführte Untersuchungen galten der Suche nach Verbindungen mit einem günstigeren Speicherungsverhältnis Läsion zu Normalskelett. Eigene Untersuchungen haben ergeben, daß aus einer großen Zahl von geprüften Präparaten, wie beispielsweise 1-Amino-1,1-ethandiphosphonsäure (A-EDP), N,N-Dimethyl-aminomethandiphosphonsäure (DMA-MDP), N-Ethyl-aminomethan-diphosphonsäure (EA-MDP), N,N-Diethylaminomethandiphosphonsäure (DEA-MDP) und N,N-Dibutylaminomethandiphosphonsäure (DBA-MDP), die zuletzt genannte Verbindung mit Abstand die höchsten Verhältnisse Q bei der Tc-99m-Speicherung in einer Läsion im Verhältnis zur Speicherung im Normalknochen aufweist (Q = (% Tc/g Läsion)/(% Tc/g Normalknochen)). Dieses Ergebnis wurde auch von Schümichen (C. Schümichen et al. Nucl. Med. 27, 8 - 11, 1988) bestätigt, der beim Vergleich der Tc-Anreicherung von DBA-MDP, DPD und dem kommerziell am häufigsten benutzten MDP in Knochenläsionen und Normalknochen die deutlich höhere Anreicherung von DBA-MDP in den Läsionen feststellte.

In der EP-A-0 096 930 wird ein für die Darstellung von Geweben geeignetes stabiles Technetium-99m Präparat beschrieben, welches aus

- Mono-, Di- oder Polyphosphonaten, die mit Tc-99m Komplexe bilden,
- einem Pertechnetat-Reduktionsmittel und
- einem Stabilisator besteht.

Das für die genannten Zwecke nach bisherigem Kenntnisstand beste Präparat ist DBA-MDP. Es weist allerdings die Nachteile einer nicht optimalen Blutclearance und dadurch eines relativ schlechten Knochen/Untergrund-Verhältnisses auf. Es besteht deshalb das Bedürfnis, bessere Präparate für die Knochenszinitigraphie insbesondere zur Erkennung von Skelettläsionen bereitzustellen.

Überraschenderweise wurde nun gefunden, daß Tc-99m-ω-Alkylphosphinico-1-hydroxyalkan-1,1-diphosphonate hervorragend für die genannten Zwecke geeignet sind.

Erfindungsgegenstand sind demzufolge Tc-99m-Präparate zur Knochenszintigraphie, die dadurch gekennzeichnet sind, daß sie mindestens eine Verbindung der Formel I enthalten,

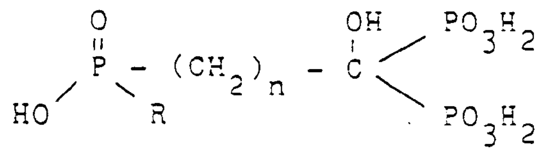

(I)

in der R eine Methyl-, Ethyl- oder Propylgruppe und n eine Zahl von 1 bis 6 ist und die in Form eines physiologisch verträglichen Salzes vorliegen kann. In der Regel enthalten die erfindungsgemäßen Tc-99m-Präparate nur eine Verbindung der Formel I oder eines ihrer Salze.

Bevorzugt sind die Verbindungen der Formel I in denen R eine Methyl- oder Ethylgruppe ist und n eine Zahl von 1 bis 3 ist. Besonders bevorzugt ist eine Verbindung, in der R eine Methylgruppe und n gleich 2 ist, d.h. die Verbindung der Formel

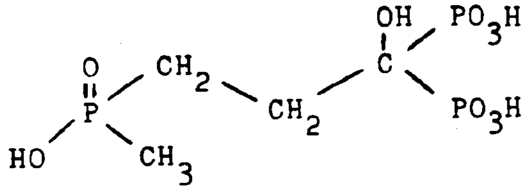

mit der Bezeichnung 1-Hydroxy-3-methylphosphinico-1,1-propandiphosphonsäure (HMPD) oder deren physiologisch verträgliche Salze. Geeignete physiologisch verträgliche Salze sind die Alkalisalze, insbesondere die Natriumsalze.

Die genannten Verbindungen haben gegenüber der Verbindung DBA-MDP, die als beste Verbindung des Standes der Technik zur Erkennung von Skelettläsionen anzusehen ist, den Vorteil, daß sie einen deutlich höheren Läsions-Quotienten Q aufweisen (vgl. Beispiel 5) und daß sie sich, wie eigene Versuche zeigten, durch eine schnellere Blutclearance auszeichnen.

Die Darstellung der erfinderisch verwendbaren Verbindungen erfolgt vorzugsweise nach dem in der deutschen Patentanmeldung P 38 05 644.5 beschriebenen Verfahren.

Weiterhin bevorzugt sind Tc-99m-Präparate, wie vorstehend beschrieben, die eine Zinn-II-Verbindung enthalten. Besonders geeignete Zinn-II-Verbindungen sind SnO und $SnCl_2$, die in einem Molverhältnis von 1:100 bis 1:2, vorzugsweise von ca. 1:20 der Verbindung der Formel I zugesetzt werden.

Der Zusatz von Zinn-II-Ionen ist erforderlich, um eine schnelle und möglichst quantitative Reduktion und Bindung des als $^{99m}TcO_4^-$ zugesetzten Radionuklids an den Wirkstoff (Diphosphonat) zu erreichen.

Es kann darüber hinaus zweckmäßig sein, dem Tc-99m-Präparat einen Stabilisator zuzusetzen. Ein besonders geeigneter Stabilisator ist N-(4-Aminobenzoyl)-glutaminsäure bzw. deren Natriumsalz, insbesondere in einem Molverhältnis von 0,5:10 bis 2:10 zur Verbindung der Formel I oder deren Salz.

Besonders geeignet ist ein Präparat, das Zinn-II, N-(4-Aminobenzoyl)-L-glutaminsäure (ABG) und 1-Hydroxy-3-methylphosphinico-1,1-propandiphosphonsäure (HMPD) im molaren Verhältnis von ungefähr

1 : 2 : 20

enthält und das, nach Markierung mit Technetium-99m, als Injektionslösung einen pH-Wert von ca. 6 - 7 aufweist.

Erfindungsgegenstand ist weiterhin ein Verfahren zur Herstellung eines Tc-99m-Präparates, das dadurch gekennzeichnet ist, daß man eine Zinn-II-Verbindung zu einer Lösung einer Verbindung der Formel I oder einem ihrer Salze gibt und das Gemisch mit einer Pertechnetat-Lösung versetzt. Zweckmäßigerweise wird die Zinn-II-Verbindung vor der Zugabe gelöst, z.B. $SnCl_2 \cdot 2H_2O$ in verdünnter Mineralsäure, z.B. Salzsäure oder Zinn-II-oxid in Alkalihydroxid, bevorzugt in Natronlauge. Die Verbindung der Formel I oder deren Salz wird ebenfalls vorzugsweise in Wasser gelöst eingesetzt. Weiterhin wird zweckmäßigerweise der Stabilisator zugegeben. Die Zugabe der einzelnen Reagenzien erfolgt in beliebiger Reihenfolge und sollte unter Luftausschluß geschehen. Die einzelnen Reagenzien sind vorzugsweise in einem derartigen Mengenverhältnis zuzugeben, daß sich ein pH von ca. 6 - 8, vorzugsweise von ca. 7 ergibt. Der Gehalt dieser Lösung an der Verbindung der Formel I oder deren Salz kann in weiten Grenzen variiert werden; er liegt vorzugsweise bei einem Gehalt von ca. 10 - 30 mg/ml Lösung.

Die wie beschrieben hergestellte Lösung wird mit Tc-99m Pertechnetatlösung, die aus einem Tc-99m-Generator gewonnen werden kann, versetzt. Ein bevorzugter Tc-99m-Generator ist in der deutschen Patentanmeldung P 35 31 355.2 beschrieben. Es ist besonders zweckmäßig, die wie beschrieben hergestellte Lösung vor der Zugabe der Tc-99m-Lösung zu lyophilisieren, vorzugsweise in einzelnen Glasgefäßen, deren Inhalt einer Markierungseinheit entspricht. Die zuletzt genannte Verfahrensweise bietet den Vorteil, daß eine über Monate haltbare und lagerfähige Markierungseinheit unmittelbar vor dem Gebrauch mit frisch eluierter Tc-99-m-Pertechneteatlösung versetzt werden kann.

Die Bestimmung der Radioaktivitätsverteilung kann mittels allgemein bekannter Apparaturen erfolgen (Gammakameras, vgl. C. Schümichen: Physiologische Grundlagen der Knochenszintigraphie; Meßtechnik und quantitative Auswertung, Der Nuklearmediziner 7, 73-88, 1984).

Durch die nachfolgenden Ausführungsbeispiele soll die Erfindung näher erläutert werden.

**Beispiel 1**

Zu einer wäßrigen Lösung von 1,134 g 1-Hydroxy-3-methylphosphinico-1,1-propandiphosphonsäuremononatriumsalzmonohydrat wurden unter Luftausschluß 40 mg $SnCl_2 \times 2H_2O$, gelöst in 1 ml 0,1 N Salzsäure, und 100 mg N-(4-Aminobenzoyl)-L-glutaminsäure mononatriumsalz, gelöst in 5 ml Wasser, zugesetzt. Die klare Lösung wurde durch Zugabe von Natronlauge auf pH 6 eingestellt und mit Wasser auf ein Gesamtvolumen von 50 ml verdünnt, sterilfiltriert und jeweils 0,5 ml, entsprechend 10 mg HMPD, abgefüllt. Die gefriergetrockneten Proben wurden durch Zugabe von Tc-99m-Generatoreluat (Pertechnetatlösung) markiert und jeweils 0,01 mg in 0,1 ml (ca 1 MBq) Ratten iv. injiziert. Die Ergebnisse der Organverteilung werden in Tabelle 2 mit denen von Beispiel 2 verglichen.

**Beispiel 2**

18,5 ml 2N Natronlauge wurden in einem 100 ml-Kölbchen vorgelegt und mit 110 mg Zinn-II-oxid versetzt, das sich bei Raumtemperatur darin vollständig löste. Unter Luftausschluß wurde zu dieser Natriumstannitlösung eine Lösung von 5,67 g 1-Hydroxy-3-methylphosphinico-1,1-propandiphosphonsäure mononatriumsalzmonohydrat und 0,45 g N-(4-Aminobenzoyl)-L-glutaminsäure (ABG) in 60 ml Wasser gegeben. Nach guter Durchmischung dieser Lösung, die einen pH-Wert von 7 aufwies, wurde mit Wasser auf ein Gesamtvolumen von 250 ml eingestellt. Die sterilfiltrierte Lösung wurde in Durchstichfläschchen vereinzelt und lyophilisiert. Jede Abfüllung (Markierungseinheit) enthielt:

10 mg HMPD,
0,2 mg $Sn^{2+}$ und
0,9 mg ABG.

**Beispiel 3**

Die Bestimmung der Markierungsausbeute von Tc-99m-HMPD und die Reinheits- und Stabilitätsprüfungen erfolgten durch dünnschichtchromatographische Methoden, Gelfiltration sowie HPLC.

1. Die Markierungsausbeute wurde durch Messung des Anteils an freiem Pertechnetat (a) und des Gehalts an reduziertem, ungebundenen Technetium (b) durch Dünnschichtchromatographie bestimmt.

a) Die Bestimmung von $^{99m}TcO_4^-$ erfolgte auf Silicagel-Glasfiberplatten (ITLC Typ SG, Hersteller Fa. Gelman, Michigan, USA) mit Methylethylketon als Fließmittel (Pertechnetat $R_f = 1$) und die von

b) reduziertem, ungebundenen Technetium ($^{99m}Tc^{4+}$) auf einer gleichen Platte mit 2 M Natriumacetatlösung als Fließmittel ($Tc^{4+}$ $R_f = 0$). Der Anteil beider Verunreinigungen in der Injektionslösung lag immer <1 %.

2. Stabilitätsprüfungen bis zu einem Zeitraum von 24 h nach Präparation erfolgten durch Gelchromatographie an Polyacrylamidgel (Bio Gel(R) P-10, Hersteller Fa. Bio-Rad, California, USA) unter Verwendung von 0,9 % NaCl-Lösung als Eluent (Abb. 1). Gemessen wurde der Aktivitätsanteil im HMPD, der als Pertechnetat vorliegende und der auf der Säule zurückbleibende (Tabelle 1). Letzterer ist größer als der Anteil an $^{99m}Tc^{4+}$, da immer eine unspezifische Adsorption von Technetium an dem Trägermaterial zu beobachten ist.

Tabelle 1

| Zeit nach Präparation | % Aktivitätsanteil im HMPD | % Tc-99m als Pertechnetat | Rückstand auf der Säule |
|---|---|---|---|
| 5 min | 92,6 | 0,6 | 6,8 |
| 2 h | 92,1 | 0,4 | 7,5 |
| 7 h | 95,0 | 0,4 | 4,6 |
| 24 h | 94,0 | 0,8 | 5,2 |

3. Die Prüfung durch HPLC (Abb. 2) zeigt ein radiochemisch reines Tc-99m-HMPD (Retentionszeit 20,5 min). HMPD (UV-Spur) unterscheidet sich von der markierten Form durch eine etwas längere Retentionszeit (21,4 min), während der unmarkierte Stabilisator ABG nach 23,4 min abgetrennt wird.

Die Daten zeigen, daß sich ein Aktivitätsanteil des Tc-99m von mehr als 90 % im HMPD befindet und daß das Präparat über einen ganzen Arbeitstag stabil ist.

**Beispiel 4**

Um die Brauchbarkeit der unterschiedlichen HMPD-Präparationen hinsichtlich ihrer Eignung zur Skelettszintigraphie zu überprüfen, wurde ihre Organverteilung in Ratten 2 Stunden nach Injektion gemessen (Tabelle 2):

Tabelle 2: Organverteilung von Tc-99m-HMPD in der Ratte (n = 3) 2 h nach i.v. Injektion in % der applizierten Testdosis pro Organ bzw. Gewebe

| | Tc-99m-HMPD | |
|---|---|---|
| | Herstellung gemäß Beispiel 1 pH 6 | Herstellung gemäß Beispiel 2 pH 7 |
| Knochen (5 %) *) | 21,3 | 31,0 |
| Leber | 0,16 | 0,17 |
| Lunge | 0,054 | 0,069 |
| Milz | 0,011 | 0,010 |
| Nieren | 0,83 | 0,77 |
| Blut (7 %) *) | 0,5 | 0,4 |
| Muskel (40 %) *) | 0,4 | 0,6 |
| Urin | - | 59,5 |
| Darm | 1,2 | 1,1 |
| Magen | 0,18 | 0,14 |
| Schilddrüse | 0,008 | 0,007 |

*) % des Körpergewichtes

Der Vergleich zeigt, daß ein Unterschied im pH-Wert des Injektionspräparates und in der Herstellungsweise (Beispiel 1 bzw. 2) zu einer deutlichen Differenz in der Speicherung im normalen Knochen führt. Für den folgenden Versuch wurde das nach Beispiel 2 hergestellte Präparat (pH = 7) benutzt.

Im Gegensatz zu anderen Diphosphonaten und auch zu Pyrophosphat wurde beim Tc-99m-HMPD keine Erhöhung der Leberspeicherung auf Kosten der Skelettakkumulation beobachtet, wenn die Substanz in größeren Mengen appliziert wurde (Tabelle 3).

**Tabelle 3:** Organverteilung von Tc-99m-HMPD in der Ratte (n = 3) 2 h p.i. in Abhängigkeit von der applizierten Menge (mg) in % der injizierten Dosis pro Organ bzw. Gewebe. Die unterschiedlichen HMPD-Mengen wurden in 0,1 ml (ca. 0,7 MBq) i.v. appliziert.

| | Applizierte Menge HMPD (mg) | | | |
|---|---|---|---|---|
| | 1 | 0,1 | 0,01 | 0,001 |
| Knochen | 28,7 | 32,6 | 34,7 | 32,9 |
| Blut | 0,8 | 0,4 | 0,4 | 0,7 |
| Muskel | 1,1 | 0,6 | 0,6 | 0,8 |
| Schilddrüse | 0,007 | 0,011 | 0,008 | 0,008 |
| Magen | 0,090 | 0,092 | 0,091 | 0,151 |
| Leber | 0,22 | 0,17 | 0,18 | 0,27 |
| Lunge | 0,079 | 0,048 | 0,048 | 0,080 |
| Nieren | 1,03 | 0,94 | 1,05 | 1,51 |
| Blase + Urin | 59,1 | 56,9 | 55,6 | 54,1 |

**Beispiel 5**

Die Prüfung von Tc-99m-HMPD am Osteosarcom-Modell erfolgte an Sprague-Dawley-Ratten. Den 7 - 12 Tage alten Tieren wurde am rechten Hinterbein ein mit $^{144}Ce$ induzierter Tumor (Delbrück 1983) paratibial implantiert. Die Untersuchung erfolgte 4 Wochen nach Tumorimplantation an insgesamt 30 Tieren.

**1. Untersuchungsablauf:** Tag 1 Tc-99m-MDP (Referenzsubstanz) Tag 3 Tc-99m-HMPD

- Injektion von je 0,01 mg Substanz i.v. in 0,05 ml mit 9,25 MBq Tc-99m
- Szintigraphie nach 1, 90 und 180 Minuten p.i.
- Speicherung der szintigraphischen Daten für die Auswertung

**2. Auswertung**

- Messung der Ganzkörperaktivität aus den szintigraphischen Daten
- Verhältnis (Q) der Flächenimpulsdichte im Tumor zu der in der contralateralen Tibia
- Vergleich des Quotienten $Q_{HMPD}$ mit dem der Präparate $Q_{MDP}$ und $Q_{DBA\text{-}MDP}$.

Die Versuchsergebnisse sind in Tabelle 4 wiedergegeben.

Tabelle 4:

| Tc-99m-Komplex | Läsions-Quotient |
|---|---|
| HMPD | 1,27 |
| DBA-MDP | 1,16 |
| MDP | 1,0 |

Der signifikant bessere Q-Wert von Tc-99m-HMPD im Vergleich zum kommerziell weitverbreiteten Tc-99m-MDP und auch zu dem bisher, aufgrund von Läsionsversuchen, als optimal angesehenen Tc-99m-DBA-MDP belegt die gegenüber den Verbindungen des Standes der Technik bessere Eignung der erfindungsgemäßen Verbindungen zur Lokalisation von Knochentumoren.

## Patentansprüche

### Patentansprüche für folgende Vertragsstaaten : AT, BE, CH, DE, FR, GB, IT, LI, LU, NL, SE

1. Tc-99m-Präparat zur Knochenszintigraphie, dadurch gekennzeichnet, daß es mindestens eine Verbindung der Formel I

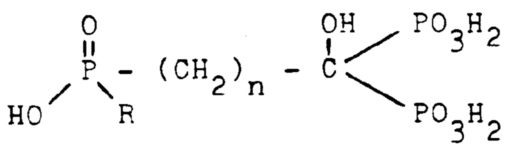

(I)

in der R eine Methyl-, Ethyl- oder Propylgruppe und n eine Zahl von 1 bis 6 ist oder mindestens eines ihrer physiologisch verträglichen Salze enthält.

2. Tc-99m-Präparat nach Anspruch 1, dadurch gekennzeichnet, daß R eine Methylgruppe und n gleich 2 ist.

3. Tc-99m-Präparat nach Anspruch 1 oder 2, dadurch gekennzeichnet, daß es eine Zinn-II-Verbindung enthält.

4. Tc-99m-Präparat nach einem oder mehreren der Ansprüche 1 - 3, dadurch gekennzeichnet, daß es eine Verbindung der Formel I oder eines ihrer Salze und die Zinn-II-Verbindung in einem Molverhältnis von 100:1 bis 2:1, vorzugsweise von ca. 20:1 enthält.

5. Tc-99m-Präparat nach einem oder mehreren der Ansprüche 1 - 4, dadurch gekennzeichnet, daß es einen Stabilisator enthält.

6. Tc-99m-Präparat nach einem oder mehreren der Ansprüche 1 - 5, dadurch gekennzeichnet, daß es als Stabilisator das Natriumsalz der N-(4-Aminobenzoyl)-glutaminsäure in einem Molverhältnis von 0,5:10 bis 2:10 zur Verbindung der Formel I oder deren Salz enthält.

7. Verfahren zur Herstellung eines Tc-99m-Präparates gemäß einem oder mehreren der Ansprüche 1 - 6, dadurch gekennzeichnet, daß man eine Zin-II-Verbindung mit einer Lösung einer Verbindung der Formel I oder einem ihrer Salze vermengt und das Gemisch mit einer Pertechnetat-Lösung versetzt.

8. Verfahren nach Anspruch 7, dadurch gekennzeichnet, daß das Gemisch aus der Zinn-II-Verbindung und der Lösung einer Verbindung der Formel I oder einem derer Salze, gegebenenfalls nach portionsweiser Abfüllung, lyophilisiert wird und vor dem Gebrauch durch Zugabe einer Tc-99m-Pertechnetat-Lösung markiert wird.

### Patentansprüche für folgende Vertragsstaaten : ES, GR

1. Verfahren zur Herstellung eines Tc-99m-Präparates, welches mindestens eine Verbindung der Formel I

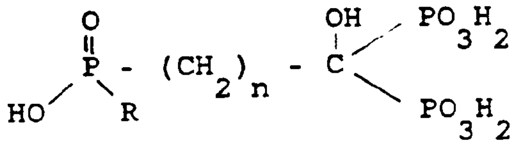

(I)

in der R eine Methyl-, Ethyl- oder Propylgruppe und n eine Zahl von 1 bis 6 ist oder mindestens eines ihrer physiologisch verträglichen Salze enthält, dadurch gekennzeichnet, daß man eine Zinn-II-Verbindung mit einer Lösung einer Verbindung der Formel I oder einem ihrer Salze vermengt und das Gemisch mit einer Pertechnetat-Lösung versetzt.

2. Verfahren nach Anspruch 1, dadurch gekennzeichnet, daß R eine Methylgruppe und n gleich 2 ist.

3. Verfahren nach Anspruch 1 oder 2, dadurch gekennzeichnet, daß das Tc-99m-Präparat eine Verbindung der Formel I oder eines ihrer Salze und die Zinn-II-Verbindung in einem Molverhältnis von 100:1 bis 2:1, vorzugsweise von ca. 20:1 enthält.

4. Verfahren nach einem oder mehreren der Ansprüche 1 - 3, dadurch gekennzeichnet, daß es einen Stabilisator enthält.

5. Verfahren nach einem oder mehreren der Ansprüche 1 - 4, dadurch gekennzeichnet, daß es als Stabilisator das Natriumsalz der N-(4-Aminobenzoyl)-glutaminsäure in einem Molverhältnis von 0,5:10 bis 2:10 zur Verbindung der Formel I oder deren Salz enthält.

6. Verfahren nach einem oder mehreren der Ansprüche 1 - 5, dadurch gekennzeichnet, daß das Gemisch aus der Zinn-II-Verbindung und der Lösung einer Verbindung der Formel I oder einem derer Salze, gegebenenfalls nach portionsweiser Abfüllung, lyophilisiert wird und vor dem Gebrauch durch Zugabe einer Tc-99m-Pertechnetat-Lösung markiert wird.

## Claims

### Claims for the following Contracting States : AT, BE, CH, DE, FR, GB, IT, LI, LU, NL, SE

1. A Tc-99m product for bone scintigraphy, which contains at least one compound of the formula I

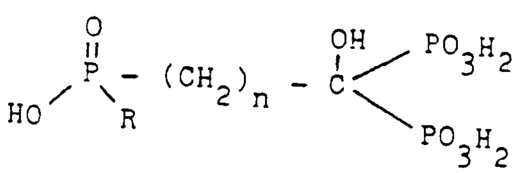

(I)

in which R is a methyl, ethyl or propyl group, and n is a number from 1 to 6, or at least one of the physiologically tolerated salts thereof.

2. A Tc-99m product as claimed in claim 1, wherein R is a methyl group and n equals 2.

3. A Tc-99m product as claimed in claim 1 or 2, which contains a tin(II) compound.

4. A Tc-99m product as claimed in one or more of claims 1 -3, which contains a compound of the formula I, or one of the salts thereof, and the tin(II) compound in a molar ratio of 100:1 to 2:1, preferably of about 20:1.

5. A Tc-99m product as claimed in one or more of claims 1 -4, which contains a stabilizer.

6. A Tc-99m product as claimed in one or more of claims 1 -5, which contains as stabilizer the sodium salt of N-(4-aminobenzoyl)glutamic acid in a molar ratio of 0.5:10 to 2:10 to the compound of the formula I, or the salt thereof.

7. A process for the preparation of a Tc-99m product as claimed in one or more of claims 1 - 6, which comprises mixing a tin(II) compound with a solution of a compound of the formula I, or one of the salts thereof, and adding a pertechnetate solution to the mixture.

8. The process as claimed in claim 7, wherein the mixture of the tin(II) compound and the solution of a compound of the formula I, or one of the salts thereof, is, where appropriate after dispensing in portions, freeze-dried and, before use, labeled by addition of a Tc-99m-pertechnetate solution.

**Claims for the following Contracting States : ES, GR**

1. A process for the preparation of a Tc-99m product for bone scintigraphy, which contains at least one compound of the formula I

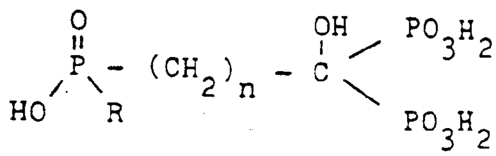

(I)

in which R is a methyl, ethyl or propyl group, and n is a number from 1 to 6, or at least one of the physiologically tolerated salts thereof, which comprises mixing a tin(II) compound with a solution of a compound of the formula I, or one of the salts thereof, and adding a pertechnetate solution to the mixture.

2. The process as claimed in claim 1, wherein R is a methyl group and n equals 2.

3. The process as claimed in claim 1 or 2, wherein the Tc-99m product contains a compound of the formula I, or one of the salts thereof, and the tin(II) compound in a molar ratio of 100:1 to 2:1, preferably of about 20:1.

4. The process as claimed in one or more of claims 1 - 3, wherein it contains a stabilizer.

5. The process as claimed in one or more of claims 1 - 4, wherein it contains as stabilizer the sodium salt of N-(4-aminobenzoyl) glutamic acid in a molar ratio of 0.5:10 to 2:10 to the compound of the formula I, or the salt thereof.

6. The process as claimed in one or more of claims 1 - 5, wherein the mixture of the tin(II) compound and the solution of a compound of the formula I, or one of the salts thereof, is, where appropriate after dispensing in portions, freeze-dried and, before use, labeled by addition of a Tc-99m-pertechnetate solution.

**Revendications**

**Revendications pour les Etats contractants suivants : AT, BE, CH, DE, FR, GB, IT, LI, LU, NL, SE**

1. Composition marquée au Tc-99m pour la scintigraphie osseuse, caractérisée en ce qu'elle contient au moins un composé de formule I

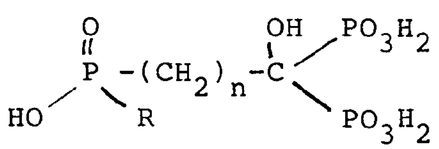

(I)

dans laquelle R est le groupe méthyle, éthyle ou propyle et n est un nombre entier allant de 1 à 6, ou au moins un de ses sels physiologiquement acceptables.

2. Composition marquée au Tc-99m selon la revendication 1, caractérisée en ce que R est le groupe méthyle et n est égal à 2.

3. Composition marquée au Tc-99m selon la revendication 1 ou 2, caractérisée en ce qu'elle contient un composé stanneux.

4. Composition marquée au Tc-99m selon une ou plusieurs des revendications 1 à 3, caractérisée en ce qu'elle contient un composé de formule I ou un de ses sels et le composé stanneux en un rapport molaire allant de 100:1 à 2:1, de préférence d'environ 20:1.

5. Composition marquée au Tc-99m selon une ou plusieurs des revendications 1 à 4, caractérisée en ce qu'elle contient un stabilisant.

6. Composition marquée au Tc-99m selon une ou plusieurs des revendications 1 à 5, caractérisée en ce qu'elle contient, en tant que stabilisant, le sel sodique de l'acide N-(4-aminobenzoyl)-glutamique en une proportion molaire allant de 0,5:10 à 2:10 par rapport au composé de formule I ou un de ses sels.

7. Procédé pour la préparation d'une composition marquée au Tc-99m selon une ou plusieurs des revendications 1 à 6, caractérisé en ce que l'on mélange un composé stanneux avec une solution d'un composé de formule I ou d'un de ses sels et on ajoute au mélange une solution de pertechnétate.

8. Procédé selon la revendication 7, caractérisé en ce que le mélange du composé stanneux et de la solution d'un composé de formule I ou d'un de ses sels, éventuellement après introduction par portions dans des récipients, est lyophilisé et marqué avant l'emploi, par addition d'une solution de Tc-99m-pertechnétate.

**Revendications pour les Etats contractants suivants : ES, GR**

1. Procédé pour la préparation d'une composition marquée au Tc-99m, qui contient au moins un composé de formule I

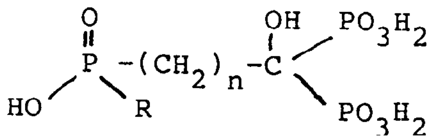

(I)

dans laquelle R est le groupe méthyle, éthyle ou propyle et n est un nombre entier allant de 1 à 6, ou au moins un de ses sels physiologiquement acceptables, caractérisé en ce que l'on mélange un composé stanneux avec une solution d'un composé de formule I ou d'un de ses sels, et on ajoute au mélange une solution de pertechnétate.

2. Procédé selon la revendication 1, caractérisé en ce que R est le groupe méthyle et n est égal à 2.

3. Procédé selon la revendication 1 ou 2, caractérisé en ce que la composition marquée au Tc-99m contient un composé de formule I ou un de ses sels et le composé stanneux en un rapport molaire allant de 100:1 à 2:1, de préférence d'environ 20:1.

4. Procédé selon une ou plusieurs des revendications 1 à 3, caractérisé en ce que la composition contient un stabilisant.

5. Procédé selon une ou plusieurs des revendications 1 à 4, caractérisé en ce que la composition contient, en tant que stabilisant, le sel sodique de l'acide N-(4-aminobenzoyl)-glutamique en une proportion molaire allant de 0,5:10 à 2:10 par rapport au composé de formule I ou un de ses sels.

6. Procédé selon une ou plusieurs des revendications 1 à 5, caractérisé en ce que le mélange du composé stanneux et de la solution d'un composé de formule I ou d'un de ses sels, éventuellement après introduction par portions dans des contenants, est lyophilisé et marqué avant l'emploi, par addition d'une solution de Tc-99m-pertechnétate.

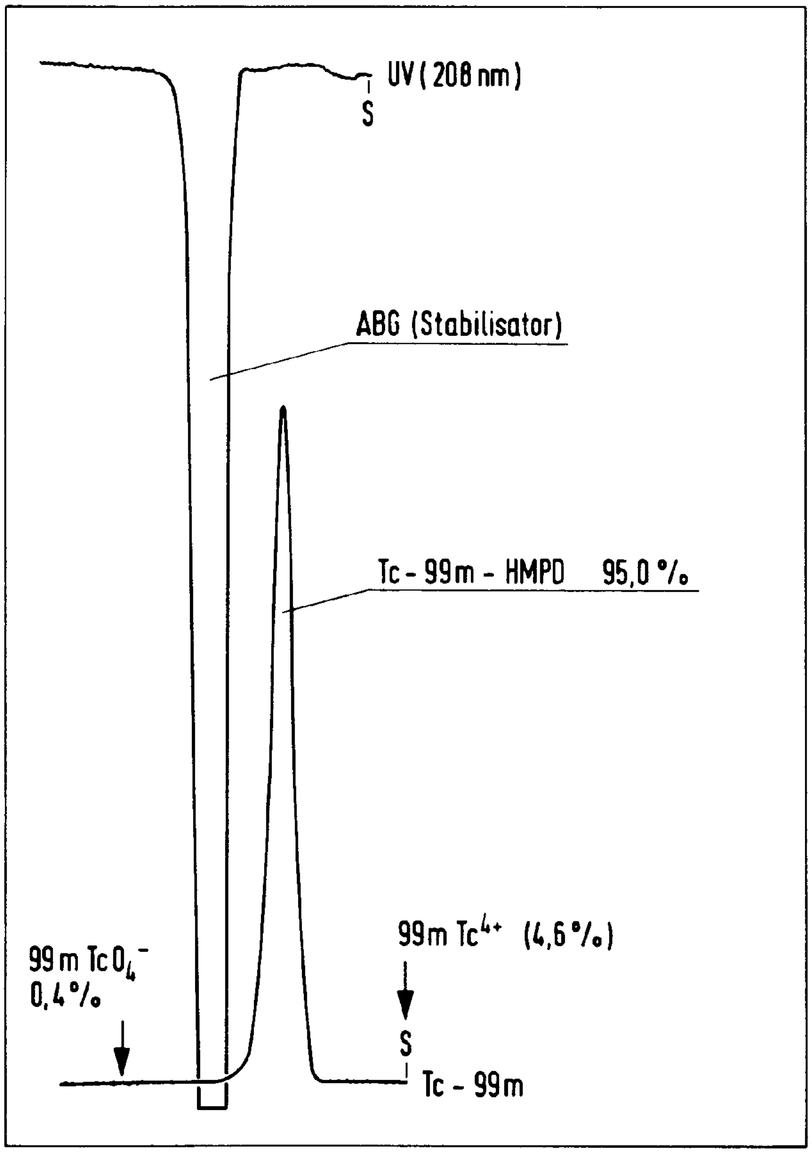


Fig. 1 Stabilitätsprüfung von Tc-99m-HMPD 7 h nach Markierung mit 3700 MBq Tc-99m-Pertechnetat in 10 ml
Säule: Bio-Gel P-10, 230 x 26mm
Eluent: 0,9 % NaCl-Lösung
Trennung von 2 ml Lösung (740 MBq)

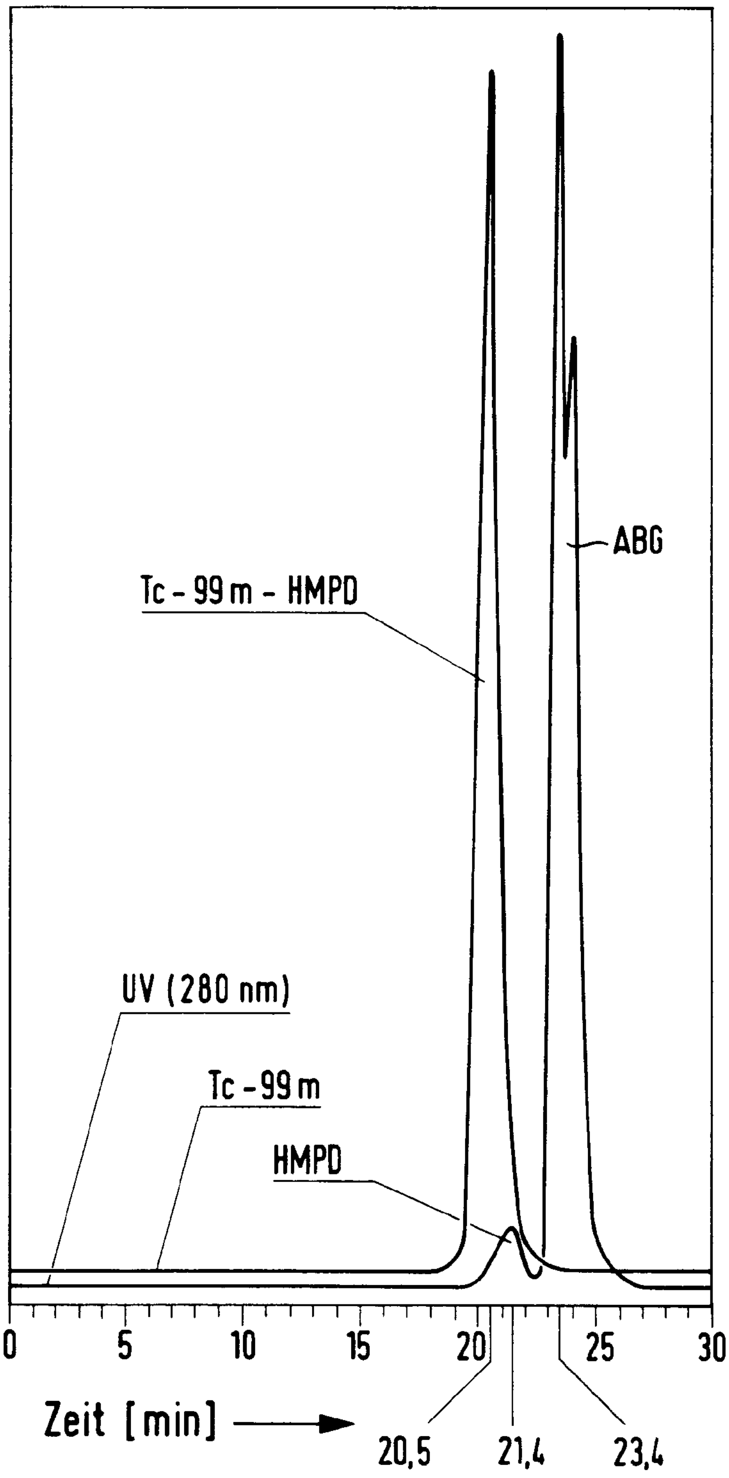


**Fig. 2** Reinheitsprüfung von Tc - 99m - HMPD durch HPLC

| | |
|---|---|
| Träger: | Bio-Rad TSK-25o, 6oo x 7,5 mm |
| Fließmittel: | o,1 M $H_3PO_4$ , pH 6,8 + o,oo5 % $NaN_3$ |
| Fluß: | 1 ml/min |
| Injektion: | o,1 ml o,1 mg (37 MBq) |